# EUROPÄISCHE PATENTANMELDUNG

(11) **EP 1 816 691 A1**
(43) Veröffentlichungstag der Anmeldung: **08.08.2007**
(21) Anmeldenummer: 07001955.9
(22) Anmeldetag: 30.01.2007
(51) Int. Cl.: H01M 2/02, A45F 5/02

(54) **Tragevorrichtung zum Mitführen einer tragbaren Energieversorgung an einem Lebewesen**

(30) Priorität: 03.02.2006 DE 202006001887 U
(71) Anmelder: ATUFORMA GmbH, 67227 Frankenthal (DE)
(72) Erfinder: Zitt, Volker, 67227 Frankenthal (DE)
(74) Vertreter: Katscher Habermann Patentanwälte

(57) **Zusammenfassung**

Bei einer Tragevorrichtung zum Mitführen einer tragbaren Energieversorgung an einem Lebewesen ist eine wiederaufladbare Batterie (2) in ein kissenförmiges Gebilde aus einem wasserundurchlässigen Kunststoffmaterial eingebettet. Das kissenförmige Gebilde ist ein Gelkissen (1). Das Gelkissen weist mindestens eine Haftfläche (6) auf. Das kissenförmige Gebilde aus einem wasserundurchlässigen Kunststoffmaterial ist mit einer Befestigungsvorrichtung verbindbar ist. Ein zu beiden Seiten überstehender Tragegurt ist in das kissenförmige Gebilde aus einem wasserundurchlässigen Kunststoffmaterial eingebettet. Der Tragegurt ist mindestens bereichsweise elastisch ausgestaltet. Ein elektrischer Verbraucher ist in das kissenförmige Gebilde aus einem wasserundurchlässigen Kunststoffmaterial eingebettet.

## Beschreibung

Die Erfindung betrifft eine Tragevorrichtung zum Mitführen einer tragbaren Energieversorgung an einem Lebewesen mit mindestens einer in einer Umhüllung angeordneten wiederaufladbaren Batterie.

Es sind beispielsweise viele medizinische oder therapeutische Anwendungen bekannt, die das Mitführen einer tragbaren Energieversorgung am menschlichen Körper über einen längeren Zeitraum erforderlich machen. So müssen oftmals bei Langzeit-Elektrokardiogrammmessungen oder anderen Langzeitmessungen von Körpereigenschaften oder -funktionen die jeweiligen Sensoren und Messvorrichtungen, die am menschlichen Körper angeordnet sind, über den gesamten Zeitraum mit Energie versorgt werden. Auch ist es oftmals zweckmäßig oder die einzige praktikable Vorgehensweise, für Langzeitstudien zu Forschungszwecken Menschen oder Tiere mit Messvorrichtungen auszustatten, die dann über einen langen Zeitraum am Körper getragen und mitgeführt und mit Energie versorgt werden müssen.

Bei bekannten Tragevorrichtungen sind eine oder mehrere wiederaufladbare Batterien in einem Gehäuse angeordnet, welches beispielsweise mit einem Tragegurt oder einer Tragegurtanordnung am Körper befestigt werden kann. Das Gehäuse kann taschenförmig ausgestaltet oder in einer Tasche angeordnet sein, um ein leichtes Auswechseln der wiederaufladbaren Batterien zu ermöglichen. Das Mitführen eines solchen Gehäuses am Körper wird allerdings oftmals als unangenehm empfunden.

Wenn die tragbare Energieversorgung auch beim Duschen, bei Regenwetter oder bei sportlicher Betätigung mitgeführt werden soll ist eine oftmals aufwändige wasserdichte Ausgestaltung des Gehäuses erforderlich, um eine Funktionsstörung oder Beschädigung der wiederaufladbaren Batterie auf Grund von eindringender Feuchtigkeit vermeiden zu können.

Aufgabe der vorliegenden Erfindung ist es demzufolge, eine Tragevorrichtung der eingangs genannten Gattung so auszugestalten, dass die Tragevorrichtung kostengünstig herstellbar und die darin angeordnete wiederaufladbare Batterie vor Feuchtigkeit geschützt ist, ohne dass damit eine nachteilige Beeinträchtigung des Tragekomforts verbunden wäre.

Diese Aufgabe wird erfindungsgemäß dadurch gelöst, dass die wiederaufladbare Batterie in einem kissenförmigen Gebilde aus einem wasserundurchlässigen Kunststoffmaterial eingebettet ist. Mit den bekannten Verfahren der Kunststoffherstellung, beziehungsweise Kunststoffverarbeitung lassen sich Kissen mit geeigneten Abmessungen aus einem wasserundurchlässigen Kunststoffmaterial schnell und kostengünstig herstellen. Das Einbetten einer wiederaufladbaren Batterie kann oftmals ohne einen zusätzlichen Verfahrensschritt während der Herstellung des kissenförmigen Gebildes aus Kunststoff erfolgen.

Das verwendete Kunststoffmaterial ist vorzugsweise ausreichend verformbar und biegsam, beziehungsweise elastisch, um sich an die Oberfläche des jeweiligen Körperbereichs anpassen und eng daran anschmiegen zu können. Es sind sehr kleine wiederaufladbare Batterien wie beispielsweise Lithiumionen-Akkumulatoren oder Lithiumpolymer-Akkumulatoren bekannt, die trotz einer hohen Speicherkapazität gegebenenfalls nur wenige Millimeter dünn sind, so dass die Tragevorrichtung mit den in dem wasserundurchlässigen Kunststoffmaterial eingebetteten wiederaufladbaren Batterien ebenfalls sehr dünn ausgestaltet sein kann. Die Tragevorrichtung kann dann unter der normalen Kleidung getragen werden, ohne aufzufallen oder für den Benutzer hinderlich zu sein.

Vorzugsweise ist vorgesehen, dass das kissenförmige Gebilde aus einem wasserundurchlässigen Kunststoffmaterial ein Gelkissen ist. Gelkissen, insbesondere Gelkissen aus einem geeigneten aushärtenden Kunststoff-Gelmaterial, weisen vorteilhafte Dämpfungseigenschaften auf und schützen die darin eingebettete wiederaufladbare Batterie nicht nur zuverlässig vor Feuchtigkeit, sondern auch dauerhaft vor übermäßiger mechanischer Beanspruchung. Gelkissen können einschließlich der darin eingebetteten wiederaufladbaren Batterie in einem einzigen Verfahrensschritt schnell und kostengünstig hergestellt werden.

Einer vorteilhaften Ausgestaltung des Erfindungsgedankens zufolge ist vorgesehen, dass das Gelkissen mindestens eine Haftfläche aufweist. Bei Verwendung eines geeigneten Gelmaterials kann eine Oberfläche oder eine Bereich einer Oberfläche des Gelkissens so ausgestaltet sein, dass eine an vielen Materialien und Oberflächen anhaftende Haftfläche entsteht. Ein derartiges Gelkissen kann ohne zusätzliche Befestigungsvorrichtungen oder Klebemittel unmittelbar auf der vorgesehenen Oberfläche, beispielsweise auf menschlicher Haut, angeordnet und leicht angedrückt werden. Auf Grund der guten Hafteigenschaften einer derartigen Haftfläche bleibt das Gelkissen wie aufgeklebt an der Oberfläche haften.

Es hat sich gezeigt, dass Haftflächen erzeugt, beziehungsweise ausgestaltet werden können, die ein zuverlässiges Befestigen eines Gelkissens auf menschlicher Haut ermöglichen und den üblicherweise auftretenden mechanischen Beanspruchungen bei einem bestimmungsgemäßen Gebrauch widerstehen können. Da sich die Hafteigenschaften einer geeigneten Haftfläche eines Gelkissens nicht verbrauchen oder mit der Zeit merklich geringer werden kann ein derartiges Gelkissen mehrfach wieder verwendet werden.

Einer Ausgestaltung des Erfindungsgedankens zufolge ist vorgesehen, dass das kissenförmige Gebilde aus einem wasserundurchlässigen Kunststoffmaterial mit einer Befestigungsvorrichtung verbindbar ist. So kann das kissenförmige Gebilde aus einem wasserundurchlässigen Kunststoffmaterial beispielsweise seitliche Ösen oder Ausformungen aufweisen, mittels derer es schnell und zuverlässig beispielsweise mit einem Tragegurt oder einer geeigneten Tragevorrichtung verbindbar ist.

Vorzugsweise ist vorgesehen, dass ein zu beiden Seiten überstehender Tragegurt in das kissenförmige Gebilde aus einem wasserundurchlässigen Kunststoffmaterial eingebettet ist. Um eine wahlweise Anordnung der Tragevorrichtung an verschiedenen Körperbereichen zu erleichtern und eine zuverlässige Befestigung mit einem hohen Tragekomfort zu ermöglichen ist vorgesehen, dass der Tragegurt mindestens bereichsweise elastisch ausgestaltet ist. Ein derartiger ringförmig ausgestalteter Tragegurt ermöglicht eine sichere und zuverlässige Befestigung der Tragevorrichtung beispielsweise sowohl an einem Arm als auch an einem Bein eines Benutzers. Zusätzliche Befestigungsvorrichtungen oder Befestigungsvorrichtungselemente wie beispielsweise Schnallen oder dergleichen sind nicht erforderlich.

Einer besonders vorteilhaften Ausgestaltung des Erfindungsgedankens zufolge ist vorgesehen, dass ein elektrischer Verbraucher in das kissenförmige Gebilde aus einem wasserundurchlässigen Kunststoffmaterial eingebettet ist. So kann beispielsweise eine elektrische Heizvorrichtung in Form von Widerstandsdrähten oder einer gedruckten Leiterbahnanordnung zusammen mit der wiederaufladbaren Batterie und einer Steuervorrichtung in das Gelkissen, beziehungsweise in das kissenförmige Gebilde aus wasserundurchlässigem Kunststoffmaterial eingebettet werden. Eine derart ausgestaltete Tragevorrichtung kann unmittelbar als Heizkissen verwendet werden. Eine gesonderte elektrische Heizvorrichtung ist dann ebenso wenig erforderlich wie elektrisch leitende Verbindungskabel und Leitungsanschlüsse an der Tragevorrichtung. Neben dem Vorteil des gesteigerten Komforts bei der Verwendung eines derartigen Heizkissens wird durch die vollständige Einbettung in das wasserundurchlässige Kunststoffmaterial ein zusätzlicher mechanischer Schutz der Heizvorrichtung einschließlich der Energieversorgung bewirkt.

An Stelle einer elektrischen Heizvorrichtung können auch andere elektrische Verbraucher eingebettet werden, so dass beispielsweise Kühlkissen oder Messvorrichtungen mit einer integrierten Energieversorgung denkbar sind.

Nachfolgend werden Ausführungsbeispiele des Erfindungsgedankens näher erläutert, die in der Zeichnung dargestellt sind. Es zeigt:
Fig. 1 eine schematische Ansicht eines Gelkissens, in welchem eine wiederaufladbare Batterie zusammen mit einer Steuervorrichtung und einer elektrischen Heizvorrichtung eingebettet sind,
Fig. 2 eine Schnittansicht des in Fig. 1 dargestellten Gelkissens längs der Linie II-II,
Fig. 3 eine schematische Darstellung einer Messvorrichtung, die zusammen mit einer wiederaufladbaren Batterie in einem kissenförmigen Gebilde aus einem wasserundurchlässigen Kunststoffmaterial eingebettet ist und
Fig. 4 eine seitliche Schnittansicht des in Fig. 3 dargestellten kissenförmigen Gebildes längs der Linie IV-IV.

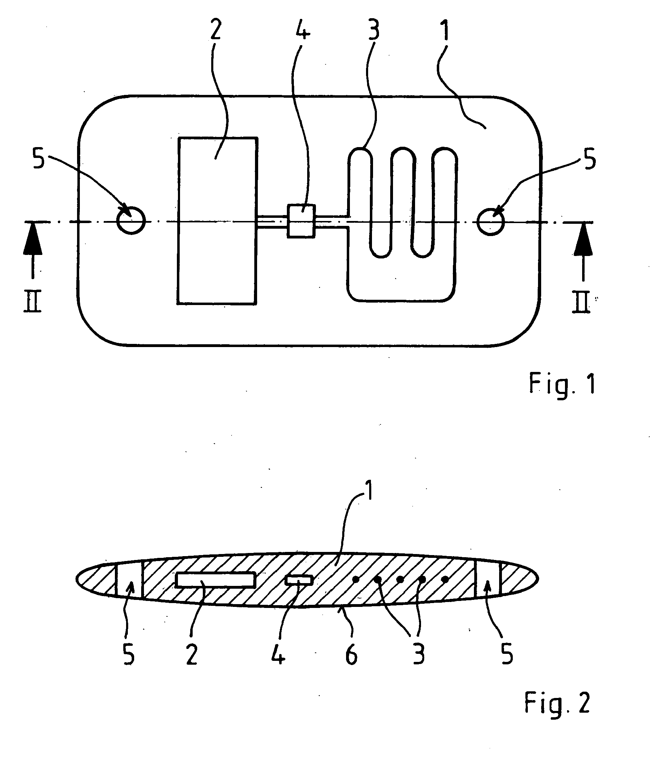

In den Fig. 1 und 2 ist schematisch ein flaches, näherungsweise handflächengroßes Gelkissen 1 dargestellt. In dem Gelkissen ist eine wiederaufladbare Batterie 2, eine damit verbundene elektrische Heizvorrichtung 3 sowie eine Steuervorrichtung 4 zur Ansteuerung der elektrischen Heizvorrichtung 3 eingebettet. In dem dargestellten Ausführungsbeispiel besteht die elektrische Heizvorrichtung 3 im Wesentlichen aus einem mäanderförmig angeordneten Heizdraht. Statt eines mäanderförmig angeordneten elektrischen Heizdrahtes können jedoch beliebige elektrische Heizvorrichtungen wie beispielsweise gedruckte Leiterbahnen beziehungsweise beliebige elektrische Verbraucher in dem Gelkissen 1 eingebettet sein.

Zum Aufladen der wiederaufladbaren Batterie 2 können entweder nicht dargestellte Anschlusskontakte bis an die Außenseite des Gelkissens 1 geführt werden oder aber eine ebenfalls nicht dargestellte Induktionsspule mit einer Gleichrichter-Schaltung mit der wiederaufladbaren Batterie 2 verbunden und in das Gelkissen 1 eingebettet sein.

Das Gelkissen 1 weist an gegenüberliegenden Seiten ösenförmige Ausnehmungen 5 auf, die zum Befestigen des Gelkissens beispielsweise an einem mit Haken versehenen Tragegurt vorgesehen sind.

Das Gelkissen weist an seiner Unterseite eine Haftfläche 6 auf, die es ermöglicht, das Gelkissen 1 in dem gewünschten Körperbereich unmittelbar auf die Hautoberfläche aufzulegen und durch leichtes Andrücken zuverlässig anhaftend mit der Haut zu verbinden.

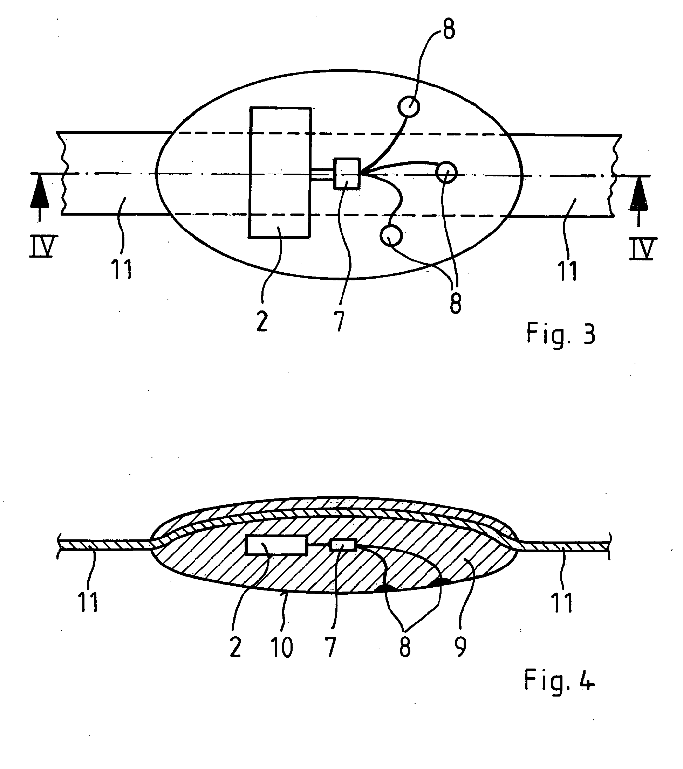

Bei dem in den Fig. 3 und 4 dargestellten Ausführungsbeispiel ist die wiederaufladbare Batterie 2 zusammen mit einer Messvorrichtung 7 und mehreren, damit verbundenen Messfühlern 8 in einem kissenförmigen Gebilde aus einem wasserundurchlässigen Kunststoffmaterial 9 eingebettet. Während die wiederaufladbare Batterie 2 sowie die Messvorrichtung 7 von allen Seiten von dem wasserundurchlässigen Kunststoffmaterial 9 umgeben sind befinden sich die Messfühler 8 an der Oberfläche 10, so dass ein unmittelbarer Kontakt mit dem menschlichen oder tierischen Körper und ein störungsfreier Messbetrieb möglich sind.

Die Tragevorrichtung weist einen Tragegurt 11 auf. Ein an die Abmessungen des kissenförmigen Gebildes aus wasserundurchlässigem Kunststoffmaterial 9 angepasster Abschnitt des Tragegurts 11 ist ebenfalls in das kissenförmige Gebilde 9 eingebettet und wird in diesem Abschnitt vollständig von dem wasserundurchlässigen Kunststoffmaterial umgeben. Auf diese Weise kann mit einfachen Mitteln eine kostengünstige und zuverlässige Befestigung des kissenförmigen Gebildes aus einem wasserundurchlässigen Kunststoffmaterial 9 mit dem Tragegurt 11 erfolgen. Der Tragegurt 11 ragt an beiden Seiten über das kissenförmige Gebilde 9 hinaus. Die zu beiden Seiten herausragenden Abschnitte des Tragegurts 11 können dazu benutzt werden, das kissenförmige Gebilde 9 an einem beliebigen Körperteil, beziehungsweise Bereich des Körpers anzuordnen, beziehungsweise anzubinden.

Der Tragegurt 11 kann an seinen beiden Enden eine Verschlussvorrichtung, beispielsweise eine Schnalle oder dergleichen aufweisen. Zur Verbesserung des Tragekomforts sowie der Handhabung ist auch denkbar, dass der Tragegurt 11 abschnittsweise oder vollständig aus einem elastischem Material hergestellt ist.

## Patentansprüche

1. Tragevorrichtung zum Mitführen einer tragbaren Energieversorgung an einem Lebewesen mit mindestens einer in einer Umhüllung angeordneten wiederaufladbaren Batterie (2), **dadurch gekennzeichnet, dass** die wiederaufladbare Batterie (2) in ein kissenförmiges Gebilde aus einem wasserundurchlässigen Kunststoffmaterial (9) eingebettet ist.

2. Tragevorrichtung nach Anspruch 1, **dadurch gekennzeichnet, dass** das kissenförmige Gebilde aus einem wasserundurchlässigen Kunststoffmaterial (9) ein Gelkissen (1) ist.

3. Tragevorrichtung nach Anspruch 2, **dadurch gekennzeichnet, dass** das Gelkissen (1) mindestens eine Haftfläche (6) aufweist.

4. Tragevorrichtung nach einem der vorangehenden Ansprüche, **dadurch gekennzeichnet, dass** das kissenförmige Gebilde aus einem wasserundurchlässigen Kunststoffmaterial (9) mit einer Befestigungsvorrichtung verbindbar ist.

5. Tragevorrichtung nach einem der vorangehenden Ansprüche, **dadurch gekennzeichnet, dass** ein zu beiden Seiten überstehender Tragegurt (11) in das kissenförmige Gebilde aus einem wasserundurchlässigen Kunststoffmaterial (9) eingebettet ist.

6. Tragevorrichtung nach Anspruch 5, **dadurch gekennzeichnet, dass** der Tragegurt (11) mindestens bereichsweise elastisch ausgestaltet ist.

7. Tragevorrichtung nach einem der vorangehenden Ansprüche, **dadurch gekennzeichnet, dass** ein elektrischer Verbraucher in das kissenförmige Gebilde aus einem wasserundurchlässigen Kunststoffmaterial (9) eingebettet ist.
